Europäisches Patentamt

European Patent Office

Office européen des brevets

(11) Numéro de publication: **0 296 359 B1**

(12) **FASCICULE DE BREVET EUROPEEN**

(45) Date de publication de fascicule du brevet: **05.08.92**  (51) Int. Cl.⁵: **A23J  3/04**, A61K 37/16

(21) Numéro de dépôt: **88107932.1**

(22) Date de dépôt: **18.05.88**

(54) **Procédé de préparation d'une composition liquide stérilisée à base de caséine.**

(30) Priorité: **09.06.87 CH 2165/87**

(43) Date de publication de la demande:
**28.12.88 Bulletin  88/52**

(45) Mention de la délivrance du brevet:
**05.08.92 Bulletin  92/32**

(84) Etats contractants désignés:
**AT BE CH DE ES FR GB IT LI LU NL SE**

(56) Documents cités:
**EP-A- 0 143 391**
**US-A- 1 962 552**
**US-A- 4 209 545**
**US-A- 4 419 369**

**FSTA 70.02.P0215 70007452: T. NAKANISHI et al.: "Studies on changes of milk casein by various treatments. VII. Effect of heat treatment on stability of casein against precipitation with calcium ions", & AGRICULTURAL AND BIOLOGICAL CHEMISTRY, vol. 33, no. 9, 1969, pages 1270-76**

(73) Titulaire: **SOCIETE DES PRODUITS NESTLE S.A.**
**Case postale 353**
**CH-1800 Vevey(CH)**

(72) Inventeur: **Stein, Jehuda**
**Zelgweg 13**
**CH-3047 Bremgarten(CH)**

Rank Xerox (UK) Business Services

## Description

L'invention concerne un procédé de préparation d'une composition liquide stérilisée à base de caséine, plus particulièrement une composition adaptée pour l'administration par voie orale ou entérale.

Pour la préparation de composition entérale stérilisée à base de caséine ayant une durée de conservation allant jusqu'à 12 mois, il faut effectuer sur ladite composition un traitement thermique de la caséine à 130°C d'au moins 3 minutes. En effet, les protéases résistant à la chaleur et contenues dans le lait précipitent lors de la préparation de la caséine acide au point isoélectrique avec ladite caséine (pH de 4,6). Tant que ces protéases ne sont pas totalement désactivées par un traitement thermique, elles provoquent une dégradation protéolytique de la caséine et induisent sur le produit final un goût amer et une coagulation au bout de 2 à 6 mois de stockage. Cette dégradation protéolytique de la caséine dépend de la concentration de protéase dans la caséine utilisée. Or, un traitement ultra-haute-température (UHT) classique de la caséine, à savoir à 148°C ou 150°C pendant 3 à 5 secondes, suffisant pour garantir une stérilité absolue, n'est pas suffisant pour inactiver complètement les faibles quantités de protéase résiduelle, difficiles à détecter analytiquement. Il faut donc avoir recours à un traitement thermique plus sévère, à savoir à 130°C pendant 5 minutes. Or, un tel traitement thermique conduit à la formation de lysinoalanine (LAL) jusqu'à 1800 mg/kg protéine et à un blocage de la lysine pouvant aller jusqu'à 20%. La présence de haute concentration de lysinoalanine (LAL) peut poser des problèmes de toxicité et le blocage de la lysine conduit à une perte nutritionnelle.

Le problème à la base de la présente invention est donc de mettre au point un procédé permettant de fabriquer une composition stérilisée à base de caséine, dans laquelle les protéases sont désactivées, ne contenant que des concentrations très faibles (150-170 mg/kg protéine) de LAL et où il n'y a pas de blocage de la lysine.

Le procédé, objet de la présente invention, permet de résoudre le problème sus-mentionné.

L'invention concerne un procédé de préparation d'une composition liquide stérilisée à base de caséine acide, dans lequel on prépare une suspension aqueuse à 10-18% en poids de caséine et une suspension aqueuse à 10-30% en poids de KOH ou NaOH et $Ca(OH)_2$, de manière à obtenir une teneur en KOH comprise entre 4 et 11 g/kg ou une teneur en NaOH comprise entre 2,5 et 8 g/kg de matière sèche de caséine et une teneur en $Ca(OH)_2$ comprise entre 14 et 20 g/kg de matière sèche de caséine, on mélange les deux suspensions pour obtenir une solution de caséinate mixte de K/Ca ou Na/Ca ayant un pH inférieur à 6,5, on traite le mélange à chaud, on refroidit, on introduit les graisses qu'on homogénéise, puis les autres additifs, on stérilise, on homogénéise et on remplit aseptiquement dans des emballages appropriés.

Le traitement thermique de caséinate s'effectue à environ 130°C pendant 5 minutes, le refroidissement à 60°-80°C et la stérilisation du produit final par UHT à environ 148°C pendant 5 secondes.

Lorsqu'on prépare la suspension de caséine, le pH est d'environ 4,6 et on effectue cette opération à une température comprise entre 10 et 65°C. Le but de l'addition de KOH ou NaOH et de $Ca(OH)_2$ est de régler le pH de la solution finale avant traitement thermique qui ne doit pas dépasser 6,5. En effet, on a constaté qu'à ce pH, la formation de LAL est très faible lors du traitement thermique subséquent. Le caséinate de potassium est soluble dans l'eau, par contre le caséinate de calcium ne l'est pas. Lorsqu'on rassemble les deux suspensions caséine (acide) et KOH ou NaOH + $Ca(OH)_2$ (alcaline), on aboutit à une solution stable colloïdale bien que le caséinate de calcium soit peu soluble dans l'eau.

En réglant bien le rapport pondéral KOH ou NaOH sur $Ca(OH)_2$, on évite toute précipitation de caséinate de calcium lors du traitement thermique et on garantit ainsi la fabrication d'un produit sans formation de dépôt pendant au moins 12 mois.

D'autre part, l'addition directe d'îons Ca sous la forme de caséinate de calcium rend inutile ou moins importante l'addition séparée de Ca sous la forme de sels insolubles, tel que $Ca(OH)_2$, $CaCO_3$ ou citrate de Ca, qui pourrait provoquer des dépôts lors du stockage, ou sous la forme de sels solubles, tel $CaCl_2$, qui peut conduire à une coagulation protéinique lors du chauffage. Comme le caséinate de calcium seul est peu soluble dans l'eau et non résistant à la chaleur, la préparation de caséinate mixte de Ca/K ou Ca/Na résistant à la chaleur permet de résoudre le problème de dépôt dans l'emballage lors d'un stockage prolongé pouvant aller jusqu'à 12 mois.

On prépare normalement selon l'invention une suspension à 10-13% en poids de caséine acide et à 10% en poids de KOH ou NaOH et $Ca(OH)_2$.

Les autres additifs qui sont ajoutés après l'addition de graisse sont les hydrates de carbone, les sels minéraux solubles tel que citrate de potassium, chlorure de magnésium, phosphate de sodium, hydroxyde de potassium, citrate, chlorure de sodium, si nécessaire, hydroxide de calcium, des oligoéléments et des vitamines.

Le fait d'ajouter les hydrates de carbone à la fin permet d'éviter le blocage de lysine, qui interviendrait si lesdits hydrates de carbone étaient présents lors du premier traitement thermique.

La suite de la description est fait en référence aux exemples mentionnant plus particulièrement la préparation de compositions pour administration par voie entérale. Les pourcentages sont en poids.

Exemple 1

Pour arriver à 1000 kg de produit final, on prépare d'un côté une suspension de 42 kg de caséine acide (correspondant à 39 kg de matière sèche) dans 400 l d'eau et d'un autre côté une suspension de 298 g de KOH et 700 g de $Ca(OH)_2$ dans 9 litres d'eau. On mélange ces deux suspensions pour arriver à une solution de caséinate mixte de K/Ca ayant un pH de 6,48. On préchauffe le mélange à 80°C et on le soumet à un traitement 130°C pendant 5 minutes, puis on refroidit brusquement à 80°C. Le pH est alors de 6,54, les graisses portées à 60°C contenant les vitamines lipo-solubles sont dosées dans le mélange, après quoi on le soumet à une homogénéisation. On refroidit ensuite à 10°C et on ajoute des hydrates de carbone (maltodextrine et syrop de glucose), des sels minéraux, des vitamines hydro-solubles et des oligoéléments. On soumet le mélange à un traitement UHT, à savoir préchauffage à 80°C, chauffage à la vapeur à 148°C pendant 5 secondes, puis refroidissement brusque à 80°C. Il reste ensuite à faire une nouvelle homogénéisation, on refroidit dans un système aseptique à 20°C, puis on charge dans une enceinte stérile pour arriver finalement sur une ligne de remplissage aseptique. Si on effectue l'analyse du produit final, on arrive aux résultats suivants:

| | | |
|---|---|---|
| pH de la composition | : | 6,72 |
| Matière sèche % | : | 19,75 |
| Matière grasses % | : | 3,39 |
| Protéines % | : | 3,78 |
| Hydrates de carbone % | : | 12,1 |
| Cendres % | : | 0,48 |
| Na | : | 47 |
| K | : | 121 |
| Ca | : | 47 |
| Mg | : | 23 |
| P | : | 48 |
| Cl | : | 95 |

mg/100 g

Teneur en lysine réactive : 7,83 g/16 g N

La teneur en LAL est de 150-170 mg/kg protéine et celle en lysine bloquée est si faible qu'elle n'est pas détectable. On ne décèle aucun dépôt après 12 mois de stockage. On n'assiste à aucun changement de structure.

Exemple 2

Pour arriver à 1000 kg de produit final, on prépare d'un côté une suspension de 42 kg de caséine acide (correspondant à 39 kg de matière sèche) dans 400 l d'eau et d'un autre côté une suspension de 213 g de KOH et 700 g de Ca $(OH)_2$ dans 9 l d'eau. On mélange les deux suspensions pour arriver à une solution ayant un pH de 6,37. Le pH de la solution après traitement à 130°C pendant 5 minutes est de 6,41. La suite des opérations de traitement sur ce mélange est la même que selon l'exemple 1.

L'analyse du produit final donne les résultats suivants:

```
pH de la composition           :    6,73
Matière sèche        %         :   21,75
Matière grasse       %         :    3,36
Protéines            %         :    6,32
Hydrates de carbone  %         :   11,5
Cendres              %         :    0,60
Na                             :   51  ⎞
K                              :  151  ⎟
Ca                             :   60  ⎬  mg/100g
Mg                             :   31  ⎟
P                              :   59  ⎟
Cl                             :   97  ⎠
Teneur en lysine réactive      :    7,53 g/16 g N
```

La teneur en LAL est faible (160 mg/kg protéine) et celle en lysine bloquée est si faible qu'elle n'est pas détectable. On ne décèle aucun dépôt après 12 mois de stockage. Il n'y a pas de changement de structure après 12 mois.

Exemple 3

Pour arriver à 1000 kg de produit final, on prépare d'un côté une suspension de 78,7 kg de caséine acide (correspondant à 72,8 kg de matière sèche) dans 400 l d'eau et d'un autre côté une suspension de 553 g de KOH et 1000 g de Ca(OH)$_2$ dans 12 l d'eau. On mélange les deux suspensions pour arriver à une solution ayant un pH de 6,18. Le pH de la solution après traitement à 130°C pendant 5 minutes est de 6,17. La suite des opérations de traitement sur ce mélange est la même que selon l'exemple 1.

L'analyse du produit final donne les résultats suivants :

pH de la composition        : 6,39
Teneur en protéines         : 6,32 %
Teneur en lysine réactive   : 7,56 g/16 g N

La teneur en LAL est faible (150 mg/kg protéine) et celle en lysine bloquée est si faible qu'elle n'est pas détectable. On ne décèle aucun dépôt après 12 mois de stockage et aucun changement de structure.

Exemple 4

Pour arriver à 1000 kg de produit final, on prépare d'un côté une suspension de 78,7 kg de caséine acide dans 400 l d'eau et d'un autre côté une suspension de 425 g de KOH et 1000 g de Ca(OH)$_2$ dans 12 l d'eau. On mélange les deux suspensions pour arriver à une solution ayant un pH de 6,12. Le pH de la solution après traitement à 130°C pendant 5 minutes est de 6,11.

La suite des opérations de traitement sur ce mélange est la même que selon l'exemple 1.

L'analyse du produit final donne les résultats suivants :

pH de la composition        : 6,33
Teneur en protéines         : 6,32 %
Teneur en lysine réactive   : 7,36 g/16 g N

La teneur en LAL est faible (150 mg/kg protéine) et celle en lysine bloquée est si faible qu'elle n'est pas détectable. On ne décèle aucun dépôt après 12 mois de stockage et aucun changement de structure.

**Revendications**

1.  Procédé de préparation d'une composition liquide stérilisée à base de caséine acide caractérisé en ce qu'on prépare une suspension aqueuse à 10-18% en poids de caséine et une suspension aqueuse à

4

10-30% en poids de KOH ou NaOH et Ca(OH)$_2$, de manière à obtenir une teneur en KOH comprise entre 4 et 11 g/kg ou une teneur en NaOH comprise entre 2,5 et 8 g/kg de matière sèche de caséine et une teneur en Ca(OH)$_2$ comprise entre 14 et 20 g/kg de matière sèche de caséine, on mélange les deux suspensions pour obtenir une solution de caséinate mixte de K/Ca ou Na/Ca ayant un pH inférieur à 6,5, qu'on traite la solution de caséinate à environ 130°C pendant environ 5 minutes, on refroidit à 60-80°C, on introduit les graisses qu'on homogénéise, puis les autres additifs, on stérilise par UHT à environ 148°C pendant 5 secondes, on homogénéise et on remplit aseptiquement.

2. Procédé selon la revendication 1, caractérisé en ce qu'on prépare une suspension aqueuse à 10-13% en poids de caséine et une suspension aqueuse à 10% en poids de KOH ou NaOH et Ca(OH)$_2$.

3. Procédé selon la revendication 1, caractérisé en ce que les autres additifs sont des hydrates de carbone, des sels minéraux solubles, des oligoéléments et des vitamines.

**Claims**

1. A process for the preparation of a sterilized liquid composition based on acidic casein, characterized in that an aqueous 10 to 18% by weight suspension of casein and an aqueous 10 to 30% by weight suspension of KOH or NaOH and Ca(OH)$_2$ are prepared so as to obtain a KOH content of from 4 to 11 g/kg or an NaOH content of from 2.5 to 8 g/kg casein dry matter and a Ca(OH)$_2$ content of from 14 to 20 g/kg casein dry matter, the two suspensions are mixed to give a solution of mixed K/Ca or Na/Ca caseinate having a pH value below 6.5, the caseinate solution is treated for about 5 minutes at approximately 130°C, cooled to 60 to 80°C, fats are introduced and homogenized, followed by the other additives, after which the product is sterilized by UHT for 5 seconds at approximately 148°C, homogenized and packed aseptically in suitable packs.

2. A process as claimed in claim 1, characterized in that an aqueous 10 to 13% by weight suspension of casein and an aqueous 10% by weight suspension of KOH or NaOH and Ca(OH)$_2$ are prepared.

3. A process as claimed in claim 1, characterized in that the other additives are carbohydrates, soluble mineral salts, oligoelements and vitamins.

**Patentansprüche**

1. Verfahren zur Herstellung einer flüssigen, sterilisierten Zusammensetzung auf der Basis von angesäuertem Casein, dadurch gekennzeichnet, daß man eine wäßrige Suspension mit 10 bis 18 Gew.-% Casein und eine wäßrige Suspension mit 10 bis 30 Gew.-% KOH oder NaOH und Ca(OH)$_2$ derart herstellt, daß man einen zwischen 4 und 11 g/kg Caseintrockensubstanz liegenden Gehalt an KOH oder einen zwischen 2,5 und 8 g/kg Caseintrockensubstanz liegenden Gehalt an NaOH und einen zwischen 14 und 20 g/kg Caseintrockensubstanz liegenden Gehalt an Ca(OH)$_2$ erhält, daß man die beiden Suspensionen so miteinander vermischt, daß man eine Lösung von gemischtem K/Ca- oder Na/Ca-Caseinat mit einem pH-Wert von unter 6,5 erhält, daß man die Caseinatlösung während etwa 5 min bei etwa 130° C behandelt, daß man auf 60 bis 80° C abkühlt, daß man die Fette einführt und homogenisiert, daß man dann die anderen Zusatzstoffe hinzufügt, daß man durch UHT (= Ultrahochtemperaturbehandlung) bei etwa 148° C während 5 s sterilisiert, daß man homogenisiert und aseptisch abfüllt.

2. Verfahren nach Anspruch 1, dadurch gekennzeichnet, daß man eine wäßrige Suspension mit 10 bis 13 Gew.-% Casein und eine wäßrige Suspension mit 10 Gew.-% KOH oder NaOH und Ca(OH)$_2$ herstellt.

3. Verfahren nach Anspruch 1, dadurch gekennzeichnet, daß die anderen Zusatzstoffe Kohlenhydrate, lösliche Mineralsalze, Spurenelemente und Vitamine sind.